# EUROPEAN PATENT SPECIFICATION

(11) **EP 0 478 680 B1**
(45) Date of publication and mention of the grant of the patent: **05.10.1994**
(21) Application number: 90910167.7
(22) Date of filing: 21.06.1990
(51) Int. Cl.: A61B 17/30

(54) **TWEEZERS FOR REMOVING TICKS**
PINZETTEN ZUM HERAUSZIEHEN VON ZECKEN
PINCES D'EXTRACTION DE TIQUES

(30) Priority: 22.06.1989 SE 8902272
(43) Date of publication of application: 08.04.1992
(73) Proprietor: IDSUND, Ake, S-123 48 Farsta (SE)
(72) Inventor: IDSUND, Ake, S-123 48 Farsta (SE)
(74) Representative: Horney, Johan
(86) International application number: PCT/SE90/00449
(87) International publication number: WO 90/15579

(56) References cited:
- AT-B- 378 116
- EXPRESSEN, 11 August 1988, STOCKHOLM, "Nu är festen slut för fästingen"

## Description

The present invention relates to tweezers intended for removing ticks from the skin, and is concerned with an improvement in tweezers of the particlar kind set forth in the preamble of Claim 1.

Tick-removing tweezers, or pincettes, of this kind differ from conventional tweezers, in that the gripping ends of said tweezers are held in mutual abutment or in abutment with the tick with an accurately predetermined spring force and the tick-tweezers, in all positions thereof, can be held in one hand and rotated by the same hand in order to twist the tick loose. Normally, the tweezers should be rotated or twisted from four to five times, so as to ensure that the head of the tick is definitely loosened from the skin. Conventional tweezers, on the other hand, can only be rotated through part of one full turn with one and the same hand grip and it is necessary to change the hand grip several times, in order to twist the tweezers through the requisite number of turns. However, when changing the grip on the tweezers, it is practically impossible not to lose the grip on the tick being removed, when using conventional tweezers. In the case of the hitherto used, known tick-tweezers according to the preamble of Claim 1, the gripping parts of the tweezer-legs are cut-off straight at the ends thereof, such as to form tweezer gripping-ends of the kind illustrated schematically and on an enlarged scale in the accompany Figures A1, A2; B1, B2; C1, C2. The cross-section of the gripping parts of the known tweezers measures 3 x 1 mm, so that the end surfaces of the gripping-ends obtain mutually the same dimensions.

Figures A1, A2 illustrate a fully grown tick A, which has a length of 3-4 mm before drinking its fill with blood. Figures B1, B2 illustrate a tick nymph, B, which in natural size has a length of 1.5-2 mm. Figures C1, C2 illustrate a larva C, which in natural size has a length of 0.5-1 mm. The width and thickness of the gripping ends of the tweezers are shown in a correspondingly enlarged scale, with a width of 3 mm.

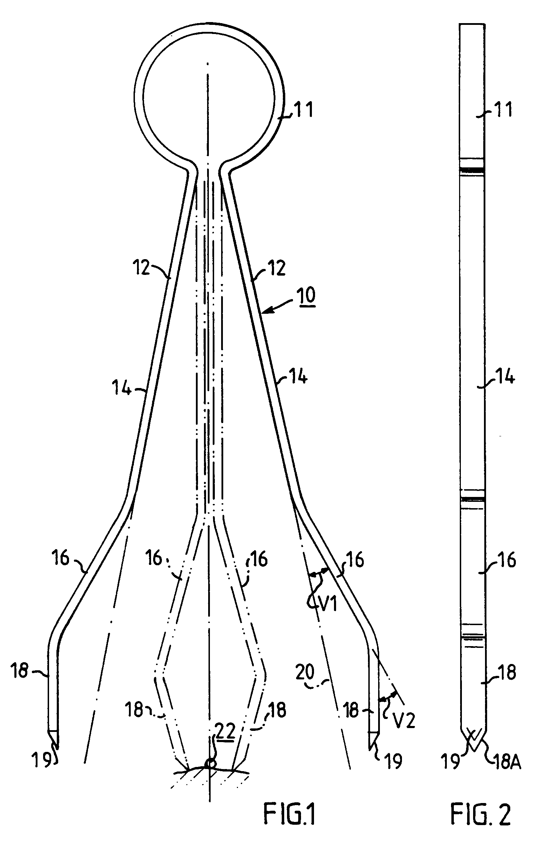

An adult tick can be seen easily, particularly after having sucked blood and therewith swelling to a size, shown in broken lines, which is several times larger than the size shown in full lines in Figures 1A, 1B, and can be gripped readily with the ends of the known tick-tweezers and twisted therewith.

People in general have long wrongly supposed that ticks are dangerous solely when fully grown and have set their teeth into a human being or an animal, particularly dogs.

Ticks, however, are not only dangerous when fully grown, but are equally as dangerous in their initial stages of development, as nymphs and larvae. Ticks develop from a larva in the first year, to a nymph in the second year and are fully-grown tick in the third year.

The dangers represented by ticks have increased in recent years and people have become more conscious of the fact that it is equally as important to be on the watch for tick nymphs as for adult ticks, since the former will bite into the skin of humans more often than the latter.

It has long been known that a bite from a fully-grown tick or from a tick nymph can result in the transmission of pathogenic bacteria, and then primarily the bacterium Borrelia. A few years ago, it was ascertained in certain districts of Sweden that one tick out of 200 was a carrier of Borrelia bacteria. Present-day surveys in the same districts show that approximately 30% of ticks are Borrelia-carriers.

In some instances, Borrelia bacteria are transferred from the mother tick to the larvae, which carry the bacteria into the nymph stage and further to the adult stage.

The result of becoming infected by a tick bite has thus increased greatly in recent years, which also applies to countries other than Sweden. The very serious symptoms of Borrelia-infection are now generally known and will not therefore be described in detail here.

The Borrelia-bacteria are found in the gastrointestinal tracts of the ticks and are transmitted to humans some days after the tick has attached itself to the skin.

Consequently, it is necessary to remove the tick as quickly and as positively as possible, irrespective of whether it is an adult, a nymph or a larva.

A fully-grown tick can, of course, be gripped securely by means of the relatively broad gripping ends of known tweezers, as illustrated in Figures A1, A2. Broad-end tweezers of this kind are also described and illustrated in Austrian Patent Specification No. 378.116, these tweezers having relatively broad gripping ends with end surfaces which are cut obliquely so as to abut one another when the tweezers are closed. The idea behind broad-end surfaces is probably to enable the body of an adult tick to be gripped more positively, prior to gripping the tweezers in a manner which will enable the tick to be twisted loose from the skin. However, although it is desireable to reach the head of the tick, this is difficult to achieve with broad gripping-ends, since such ends will not readily penetrate the skin. Consequently, the legs of the tweezers are normally clamped against the tick's body, particularly when it is filled with blood. This increases the risk of wrenching the tick's body from its head and of leaving the head embedded in the skin, with the subsequent risk of infection. It is almost impossible to obtain a grip on a tick nymph when using broad-ended tweezers.

Consequently, it is impossible, in the majority of cases, to use the known tick-tweezers for the purpose of removing tick nymphs which are much smaller, length 1.5-2 mm, than fully-grown ticks, length 3-4 mm or much longer when filled with blood. These known tick-tweezers are practically unusable for removing tick larvae.

Knowing that it is usually tick nymphs which bite into people, and also being aware of the fact that ticks carry pathogenic bacteria, it is evident that there is a need for tick-tweezers which, in comparison with the known tick-tweezers, can be used as a precision instrument for removing adult ticks, nymphs and larvae.

This need is satisfied with tweezers constructed in accordance with the present invention and having the characteristic features set forth in the following Claim 1.

Tweezer legs which taper down to a point at the gripping ends thereof are, of course, well known from conventional tweezers. However, in combination with the specific design of the rotatable tick-tweezers according to the preamble of Claim 1, there is obtained a novel type of tick-tweezers which can be used by any one whomsoever to remove readily and positively such small creatures as tick nymphs and tick larvae with great precision, it being possible to hold the tick-tweezers in one hand and to rotate the tweezers with the aid of said one hand with the tick, nymph or larva held firmly clamped by the tweezers, similar to the earlier known tweezers.

It is appropriate to use a magnifying glass when removing nymphs or larvae, so that the pointed ends of the tweezers can be placed accurately on a fully-grown tick, a nymph or a larva, closely contiguous therewith.

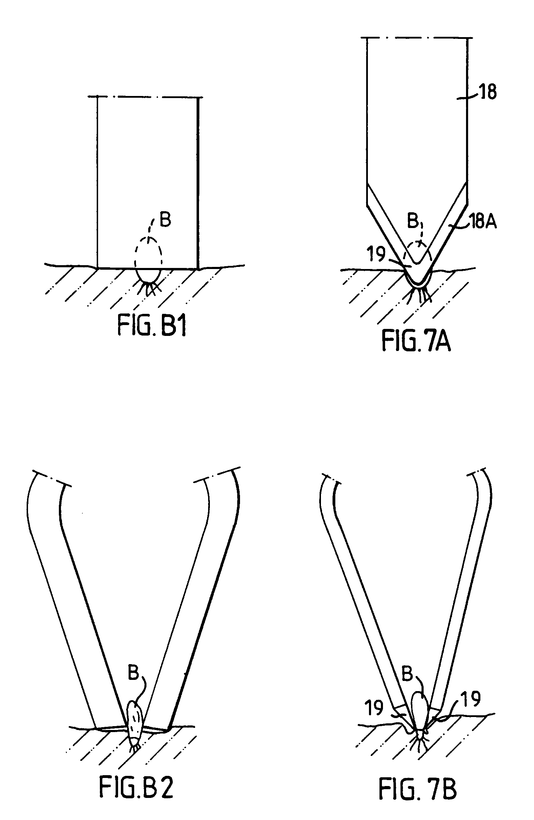

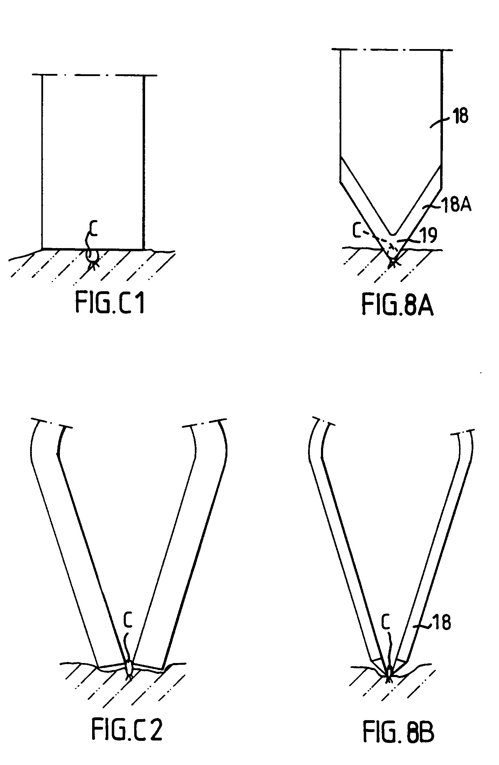

Accurate alignment and engagement of the pointed tweezer-ends with the nymph (Figure 7A) or the larva (Figure 8A) is greatly facilitated, in accordance with the invention, since vision of the nymph or larva is not obstructed by broad gripping ends, as in the case of the known tick-tweezers (Figures B1, C1). Furthermore, the gripping ends are only half as thick, 0.5 mm, as the known gripping ends, 1 mm.

Since the gripping ends are pointed and relatively thin, the ends of the tweezers can be pressed into the skin on the sides of, e.g. the nymph or the larva, more readily than was previously the case, therewith to obtain a more positive grip against the head of the tick or nymph, since the known, broad gripping ends will, of course, meet with greater resistance when being pressed into the skin.

A preferred, exemplifying embodiment of the invention is illustrated in the accompanying drawings.

The known tick-tweezers are illustrated in Figures A-C by way of comparison, wherein
Figures A1 and A2 illustrate, on an enlarged scale, an adult tick, shown from the front and from the side respectively, having a length of about 4 mm and having attached itself to the skin and being gripped with the broad and thick grippings ends, 3 x 1 mm of the known tweezers;
Figures B1 and B2 are views of the known gripping ends similar to the view of the previous Figures, and show said ends gripping a tick nymph, length 1.5-2 mm; and Figures C1 and C2 are views of the known gripping ends similar to the views of the preceding Figures and show said ends gripping a tick larva, length 0.5-1 mm.

The constructional configuration of the selected exemplifying enmbodiment of the invention is illustrated in the following Figures:
Figure 1 is a front view of the legs of the inventive tweezers in a freely, outwardly-sprung state and in an intermediate state, shown in chain lines, in which the pointed ends of the gripping parts of the tweezers are still spaced apart, prior to the tweezer legs finally springing together.
Figure 2 is a side view illustrating the flat and thin tweezer legs, which have a size of 0.5 x 3 mm.
Figure 3 shows, in section, the tweezer-arms provided with a cylindrical and helical thrust spring and a tweezer-manipulating sleeve, with the gripping ends of the tweezers in mutual abutment with a predetermined clamping force, and also shows, in chain lines, the gripping parts spaced apart, subsequent to drawing back the sleeve.
Figure 3A is a sectional view taken on the line 3A-3A in Figure 3.
Figure 4 illustrates in an enlarged scale, a fully-grown tick with the head of the tick buried beneath the skin and the body of the tick partially filled with blood, the tick being shown gripped with the gripping parts of the tweezers.
Figure 5 is an illustration similar to that of Figure 4, but with the body of the tick torn away, the pointed gripping-ends of the tweezers having been pressed down into the skin, on either side of the head, and released so as to be brought into clamping engagement with said head, prior to twisting the head loose.
Figures 6A, 6B are illustrations similar to Figures A1, A2, but show the inventive pointed and thin gripping-ends in engagement with a fully-grown tick.
Figures 7A, 7B are illustrations similar to Figures B1, B2, but show the inventive pointed gripping-ends in engagement with a nymph; and
Figures 8A, 8B are illustrations similar to Figure C1, C2, but show the inventive pointed gripping-ends in engagement with a larva.

As illustrated in Figures 1 and 2, a spring blade, measuring 0.5 x 3 mm, is bent to a tweezers-configuration, comprising two sprung legs 10 and 12 mutually joined by an oval or substantially circular end-part 11 capable of being supported against the palm of one hand and rotated with the fingers in a convenient fashion.

Since the legs 10 and 12 are mutually identical, only the leg 10 will be described in the following.

The leg 10 is bent to include three leg-parts, namely a substantially straight main part 14, a substantially straight press-part 16, and a substantially straight gripping-part 18 terminated with a pointed gripping-end 19. The gripping-end 19 has a flat inner surface, out to the edges of said end, and is cut obliquely so that the end-part of the gripping-part obtains converging side edges, so as to form said pointed end. The side edges 19A are bevelled down to the edges of the flat inner surface. For the sake of clarity, it is mentioned that the knife-like side-edges are somewhat blunt in reality, so as not to form cutting edges. This also applies to the pointed ends 19, the tips of which are slightly rounded, so as not to puncture the skin. As will be understood, puncturing of the skin is highly undesirable, since blood present beneath the skin may then come into contact with the tick and be contaminated with bacteria carried thereby.

The angle V1 formed between the main part 14 and the outwardly bent press-part 16 of respective legs is in the order of 15 to 25°, and the angle V2 between the press-part 16 and the inwardly folded gripping-part 18 is in the order of 25-35°.

As illustrated in Figure 1, the gripping end 19 is in spaced relationship with a line 20 which forms a geometric extension of the inner surface of the main part 14, whereby the pointed ends 19 will be mutually spaced apart when the main parts 14 of respective legs are in mutual abutment, as illustrated in chain lines.

In order to bring the ends 19 closer together, or into abutment with one another, it is necessary to press on the press-part 16, in order to bring the gripping parts into clamping engagement with the body of the schematically shown adult tick 22 in Figure 1.

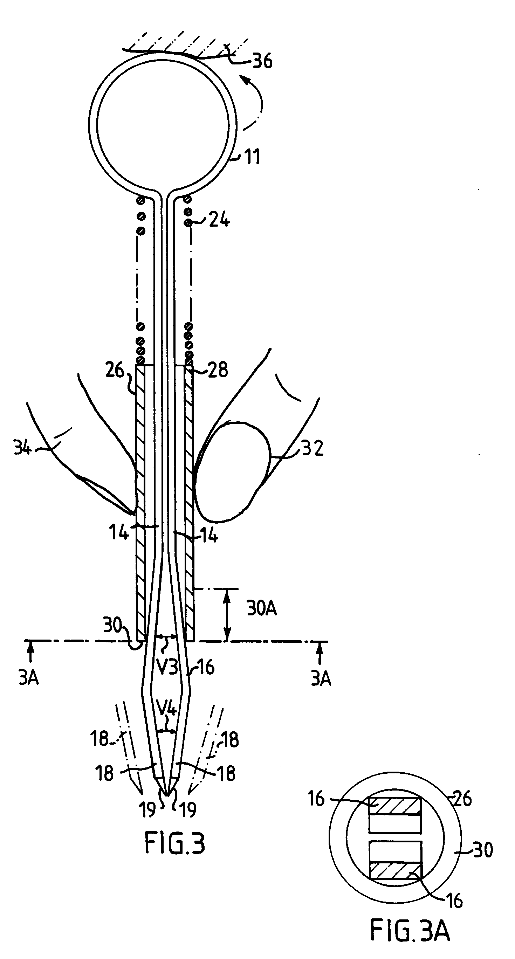

The tweezers illustrated in Figure 3 include the two sprung legs 10 and 12 of the Figure 1 embodiment, and also include a cylindrical, helical thrust spring 24 and a cylindrical tweezer-manipulating sleeve 26 which are moved over the legs 10 and 12 when said legs are brought together. The spring lies pre-tensioned between the end part 11 and the rear end 28 of the sleeve, whereas the front end-edge, 30, of the sleeve of the embodiment illustrated in Figures 3 and 3A, slides along the pressure parts 16 such as to bring the ends 19 into sprung engagement with each other at a given force determined by the spring 24, the spring force in the main parts 14 which are clamped in a mutually adjacent position, the angle V3 between the partially collapsed pressure part 16, and the friction between the edges of said press-parts and the front end-edge of the sleeve is between 20 and 30°. The angle V4 between the mutually compressed gripping-parts of the Figure 3 embodiment lies roughly between 10 and 20° where the sleeve is unable to press the gripping parts together to a greater extent than that shown in Figure 3.

It has been found that when the distance between the ends 19 is 1 mm, the clamping force shall be about 100 g, in order to prevent the tick from being subjected to excessively large clamping forces such as to squash the tick and force out its stomach contents at the place where the tick has burrowed into the skin.

Slight withdrawal 30A of the sleeve will cause the edge of said sleeve to slide down along the gripping-parts and permit said parts to spring out to the open position illustrated in chain lines. When the sleeve is released, it is moved back by the pressure spring to the illustrated end position, while bringing the gripping-parts together against the action of the spring force in the press-parts. If the main parts 14 should extend straight to the gripping parts, instead of via the outwardly bent press-parts, it would be necessary to move the sleeve through a longer distance in order to open the gripping-parts to the same extent, while the frictional engagement between the main-parts and the end-edge of the sleeve would be greater and thus constitute greater resistance to displacement of the sleeve in a direction away from the front terminal position of said sleeve.

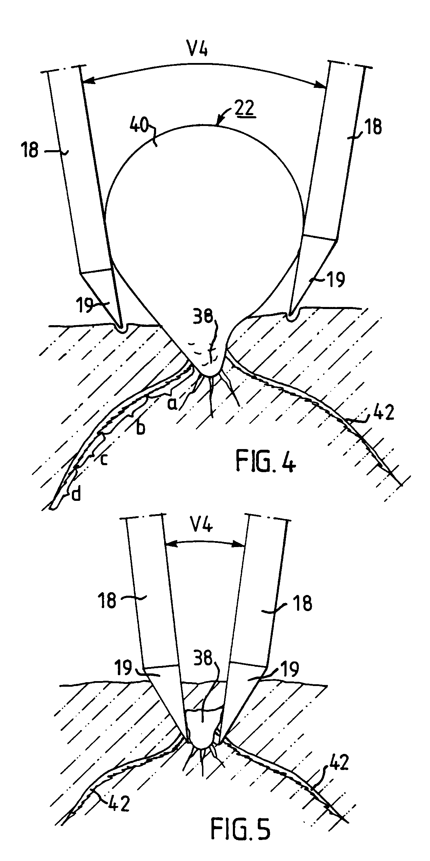

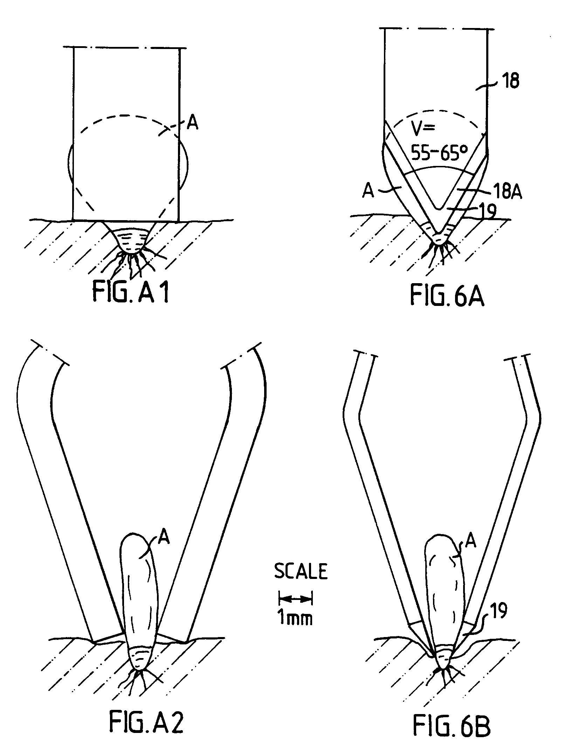

As illustrated in Figure 3, the sleeve is gripped between the thumb 32 and the index finger 34, while pressing the end-part 11 against the palm 36 of the hand. The sleeve can now be readily withdrawn slightly, in order to open the gripping-parts and then released so that said gripping parts will return automatically to their clamping position in which the body of the tick, or the blood-filled body thereof is firmly clamped as shown in Figure 4. Figures 6A and 6B illustrate how the gripping-ends grip firmly against an adult tick A which has not yet become bloated with blood, in the vicinity of the head of said tick. Figures 7A and 7B illustrate schematically how the gripping-ends 19 can be brought into good gripping engagement with the illustrated nymph B, in the vicinity of its head. Figures 8A and 8B show how the pointed gripping-ends 19 can be used to positively grip a body as small as a tick larva C.

The sleeve and the legs 10, 12 are then twisted or rotated through a desired number of turns, normally from four to five turns, preferably in an anticlockwise direction, so as to twist loose the head of the tick, therewith enabling the thus loosened tick to be withdrawn from the skin.

Figure 4 illustrates a blood-filled tick in greatly enlarged scale, and shows the head 38 of the tick, with a number of jaws, and the body 40 of said tick. Appended on the head 2 are a number of hooked arms 42 buried beneath the skin. The head of the tick is also buried beneath the skin.

The body of the tick is gripped with the gripping parts 18, which define an angle therebetween and which mutually abut one another with a carefully adapted clamping force such as to release their engagement with said body when an attempt is made to withdraw the tick without first twisting its head through a number of turns. When the tweezers are rotated by means of the manipulating sleeve, the legs 42 will be wound or twined around the head through a distance a during the first turn, and through a distance b, a distance c and a distance d during respective following turns. At the same time, the jaws on the head of the tick will be released and, in the majority of cases, the grip of the tick will have been released at least after the last turn made by the tweezers.

Figure 5 illustrates schematically the case where the body of a tick has been loosened by attempting to loosen the tick by withdrawing it with the fingers, without rotating the tick. The head of the tick is left beneath the skin in a not-readily reached location, although it can be gripped relatively easily with the pointed gripping-ends 19 of the inventive tweezers, whereafter the tweezers are carefully rotated, by twisting the manipulating sleeve. In this instance, the tweezers function as a simple and effective precision instrument for carrying out the operation concerned, an operation which is practically impossible to carry out when using the broad and thick gripping ends of known tick-tweezers.

Figure 6A illustrates how the pointed gripping-ends of the inventive tweezers can be moved down into the vicinity of the illustrated tick A, the body of which is still empty of blood. Figure 6B shows that the fully-grown tick is relatively flat, before its body is filled with blood.

Figure 7A shows that the pointed ends of the tweezers will not obstruct the illustrated nymph B, when the ends are to be positioned accurately on both sides of the nymph.

Figure 8A shows how the pointed ends of the inventive tweezers can be positioned with great precision on each side of a tick larva C, an important advantage in this respect being that the larva is not hidden from view by broad gripping-ends.

## Claims

1. Tweezers for removing ticks which have bitten into the skin, said tweezers comprising two flat, sprung legs (10, 12) which lie in the vicinity of one another at one end thereof and which merge with one another through a curved end part (11), and the free ends of which legs are configured with gripping-ends (19) which can be brought into mutual abutment by collapsing the legs against the action of their spring force, wherein each leg (10, 12) is pre-bent such as to obtain a long, straight main-part (14) which departs from said end-part (11) and the other end of which merges with an outwardly bent, substantially straight press-part (16) having a width of about 3 mm and forming an angle (V1) of 15-25° with said main part, said press-part (16) then merges with a substantially straight, inwardly folded gripping-part (18) which forms an angle (V2) of 25-35° with said press-part (16) and the free end of which is configured with a gripping-end (19) which has a flat inner surface and which, in the free state of the legs (10, 12) lies in spaced relationship with a line (20) which constitutes an extension of the inner surface of the straight main-part, such that when said straight main-parts are brought together, it is necessary to spring the gripping-parts (18) inwardly in order to bring the gripping-ends (19) into mutual abutment, and said tweezers further including a cylindrical, helical thrust spring (24) and a cylindrical manipulating sleeve (26) which is displaced inwardly on the legs (10, 12) so that the spring (24) is pre-tensioned between the rear edge (28) of the sleeve and the end-part (11) of said legs, and the front edge (10) of said sleeve lies against the outwardly bent press-parts (16) and exerts thereon a pressure force of such size that the press-parts are brought bendingly together such that the gripping-ends (19) will abut one another with a predetermined clamping or gripping force, and wherein the angle (V3) defined between the sprung, outwardly-bent press-parts (16), when the gripping-ends (19) are in mutual abutment, is between 20 and 30°, at the same time as the angle (V4) between the gripping-parts (18) is between 10 and 15°, **characterized** in that the side-edges (18A) of each gripping-part (18) converge to a pointed gripping-end (19); and in that said pointed side-edges are bevelled on the outside surface thereof down to the side-edge of the flat inner surface of each gripping-part, so that the pointed ends (19) can be used to grip a relatively large, adult tick, a relatively small tick nymph, or a relatively small tick larva.

2. Tweezers according to Claim 1, **characterized** in that when the distance between the pointed ends is kept at 1 mm, the gripping force between said ends is about 100 g.

3. Tweezers according to Claim 1 or 2, **characterized** in that the thickness of the gripping-parts is about 0.5 mm.

## Patentansprüche

1. Pinzette zum Herausziehen von Zecken, die sich in der Haut festgebissen haben, wobei die Pinzette besteht aus Zwei flachen, gefederten Schenkeln (10, 12), die an einem ihrer Enden benachbart zueinander angeordnet sind und die über einen gebogenen Endbereich (11) ineinander übergehen, und wobei die freien Enden der Schenkel mit Greifenden (19) versehen sind, die in gegenseitige Anlage bringbar sind, indem die Schenkel entgegen der Wirkung ihrer Federkraft zusammengedrückt werden, wobei jeder Schenkel (10, 12) derart vorgebogen ist, daß man einen langen, geraden Hauptbereich (14) erhält, der von dem Endbereich (11) ausgeht und dessen anderes Ende in einen nach außen gebogenen, im wesentlichen geraden Druckbereich (16) übergeht, der eine Breite von ungefähr 3 mm aufweist und mit dem Hauptbereich einen Winkel (V1) von ungefähr 15° bis 25° einschließt, wobei der Druckbereich (16) in einen im wesentlichen geraden, nach innen umgebogenen Greifbereich (18) übergeht, der mit dem Druckbereich (16) einen Winkel (V2) von 25° bis 35° einschließt und dessen freies Ende mit einem Greifende (19) versehen ist, das eine flache innere Oberfläche aufweist und das im losgelassenen Zustand der Schenkel (10, 12) beabstandet von einer Linie (20) liegt, die eine Verlängerung der inneren Oberfläche des geraden Hauptbereiches bildet, so daß es beim Zusammenbringen der geraden Hauptbereiche notwendig ist, die Greifbereiche (18) nach innen zu biegen, um die Greifenden (19) in gegenseitige Anlage zu bringen, und wobei die Pinzette außerdem eine zylindrische Druckspiralfeder (24) und eine zylindrische Betätigungshülse (26) enthält, die mit ihrer Innenseite auf den Schenkeln (10, 12) verschiebbar ist, so daß die Feder (24) zwischen der rückwärtigen Kante (28) der Hülse und dem Endbereich (11) der Schenkel vorgespannt ist, und wobei die vordere Kante (10) der Hülse gegen die nach außen gebogenen Druckbereiche (16) in Anlage befindlich ist und hierauf eine Druckkraft von einer solchen Größe ausübt, daß die Druckbereiche aneinander gebogen werden, so daß die Greifenden (19) mit einer vorbestimmten Klemm- oder Greifkraft in Anlage gebracht werden, und wobei der Winkel (V3), der bei gegenseitiger Anlage der Greifenden (19) zwischen den gefederten, nach außen gebogenen Druckbereichen (16) gebildet wird, zwischen 20° und 30° beträgt, wobei gleichzeitig der Winkel (V4) zwischen den Greifbereichen (18) zwischen 10° und 15° beträgt,
dadurch gekennzeichnet,
daß die seitlichen Kanten (18A) jedes Greifbereiches (18) in einem spitzen Greifende (19) zusammenlaufen, und daß die zugespitzten Seitenkanten an ihrer äußeren Oberfläche abgeschrägt sind bis zu der Seitenkante der flachen inneren Oberfläche jedes Greifbereiches, so daß die spitzen Enden (19) dazu verwendet werden können, eine relativ große, erwachsene Zecke, eine relativ kleine Zeckenpuppe oder eine relativ kleine Zeckenlarve zu greifen.

2. Pinzette nach Anspruch 1,
dadurch gekennzeichnet,
daß bei einem Abstand zwischen den Spitzenenden von 1 mm die Greifkraft zwischen den Enden ungefähr 100 g beträgt.

3. Pinzette nach einem der Ansprüche 1 oder 2,
dadurch gekennzeichnet,
daß die Dicke der Greifbereiche ungefähr 0,5 mm beträgt.

## Revendications

1. Pincettes pour retirer des tiques qui ont pénétré dans la peau, ces pincettes comprennant deux branches plates à ressort (12, 10) qui s'étendent au voisinage l'une de l'autre à l'une de leurs extrémités et qui se raccordent mutuellement par une partie d'extrémité courbe (11), les extrémités libres de ces branches ayant la configuration d'extrémités de préhension (19) qui peuvent être amenées en contact mutuel en rapprochant les branches contre l'action de leur force de ressort, dans lesquelles chaque branche (10, 12) est pré-courbée de façon à obtenir une partie principale longue et rectiligne (14) qui part de la partie d'extrémité précitée (11), et dont l'autre extrémité se raccorde avec une partie de pression (16), pratiquement rectiligne et courbée vers l'extérieur, ayant une largeur d'environ 3 mm et formant un angle (V1) de 15-25° avec la partie principale, cette partie de pression (16) se raccordant ensuite avec une partie de préhension (18), pliée vers l'intérieur et pratiquement rectiligne, qui forme un angle (V2) de 25-35° avec la partie de pression (16), et dont l'extrémité libre présente la configuration d'une extrémité de préhension (19) qui a une surface intérieure plane et qui, dans l'état libre des branches (10, 12), est espacée vis-à-vis d'une ligne (20) qui constitue un prolongement de la surface intérieure de la partie principale rectiligne, ce qui fait que lorsque les parties principales rectilignes sont amenées l'une contre l'autre, il est nécessaire de déformer les parties de préhension (18) vers l'intérieur, de façon élastique, pour amener les extrémités de préhension (19) en contact mutuel, et ces pincettes comprennent en outre un ressort de poussée (24) cylindrique et hélicoïdal, et une douille de manipulation cylindrique (26) qui est déplacée vers l'intérieur sur les branches (10, 12), de façon que le ressort (24) soit pré-comprimé entre le bord arrière (28) de la douille et la partie d'extrémité (11) des branches, et le bord avant (10) de cette douille porte contre les parties de pression (16) courbées vers l'extérieur, et il exerce sur elles une force de pression d'une valeur telle que les parties de pression sont amenées l'une contre l'autre avec une action de flexion, de façon que les extrémités de préhension (19) portent l'une contre l'autre avec une force de préhension ou de serrage prédéterminé, et dans lesquelles l'angle (V3) qui est défini entre les parties de pression (16) courbées vers l'extérieur de façon élastique, lorsque les extrémités de préhension (19) portent l'une contre l'autre, est compris entre 20 et 30°, et simultanément l'angle (V4) entre les parties de préhension (18) est compris entre 10 et 15°, **caractérisées** en ce que les bords latéraux (18A) de chaque partie de préhension (18) convergent vers une extrémité de préhension pointue (19); et en ce que les bords latéraux pointus précités sont biseautés sur leur surface extérieure, en descendant jusqu'au bord latéral de la surface intérieure plane de chaque partie de préhension, de façon que les extrémités pointues (19) puissent être utilisées pour saisir une tique adulte, relativement grande, une nymphe de tique relativement petite ou une larve de tique relativement petite.

2. Pincettes selon la revendication 1, **caractérisées** en ce que lorsque la distance entre les extrémités pointues est maintenue à 1 mm, la pression de préhension entre ces extrémités est d'environ 1 N.

3. Pincettes selon la revendication 1 ou 2, **caractérisées** en ce que l'épaisseur des parties de préhension est d'environ 0,5 mm.
